# EUROPEAN PATENT APPLICATION

(11) **EP 1 974 773 A2**
(43) Date of publication of application: **01.10.2008**
(21) Application number: 08251236.9
(22) Date of filing: 31.03.2008
(51) Int. Cl.: A61Q 19/04, A61K 8/55

(54) **Compositions for use in darkening the skin**

(30) Priority: 29.03.2007 US 693023
(71) Applicant: Johnson & Johnson Consumer Companies, Inc., Skillman, NJ 53008 (US)
(72) Inventor: Lin, Connie B., Belle Mead, NJ 08502 (US); Seiberg, Miri, Princeton, NJ 08540 (US)
(74) Representative: Kirsch, Susan Edith

(57) **Abstract**

The present invention features glycerophospholipids having a single fatty acid moiety and the use thereof in darkening the skin.

## Description

### FIELD OF THE INVENTION

The present invention relates to glycerophospholipids having a single fatty acid moiety, such as lysophosphatidylcholine, and the use thereof to darken the skin.

### BACKGROUND OF THE INVENTION

Many individuals desire the darkening of skin color. Most people obtain darker skin through exposure to UV light (e.g., suntanning or UV lamps). Production of melanin and the type of melanin when stimulated by UV are genetically determined. UV exposure, however, results in accelerated skin aging and increased incidence of skin cancer. The ability to generate a tanned appearance without incurring photodamage, thus, is important to many individuals. Accordingly, alternative methods for "sunless tanning" have evolved.

One method is the use of products containing dihydroxy acetone (DHA). These products, however, produce color that is too orange and unnatural to the user. Moreover, the DHA-produced skin color only minimally protects the user from UV irradiation. Products containing beta-carotene, cantaxanthin and lycopene have also been used to darken the skin. These products, however, have no effect at all on melanogenesis and usually result in unnatural and uneven skin color by staining the fat layers just below the skin. In addition, these products do not provide any sun-protection as compared to naturally tanned skin.

Melanotan and MelanX are synthetic hormone drugs that mimic the action of melanocyte-stimulating hormone (MSH) and are used to darken the skin when administered by injection. These agents are not topically active, due to their poor or lack of skin permeation, therefore they require medical administration, and consequently are very costly. Moreover, these agents are regulated and are not approved in many countries. Another method to darken the skin uses psoralens, which are photo-sensitizers and therefore enhance melanin production when combined with UV exposure. Psoralens do not darken the skin without UV exposure, which must be carefully regulated to minimize the risk of skin cancer. Psoralens, in conjunction with medical grade UV lamps, are accepted as treatment for people afflicted with vitiligo and psoriasis, but are not recommended for patients with fair skins for tanning.

Thus, a product is desired that is safe and inexpensive, that would enhance the body's natural pigment content, resulting in a desired skin color and enhanced photo-protection, without the need of UV exposure, and without the need of medical intervention.

Phospholipids, lecitins and lysophosphatidylcholine (LPC) are used in many skin care products as moisturizers, topical carriers, and/or anti-aging agents, and they are typically associated with products for skin lightening. However, LPC has been reported to mediate *in vitro* certain processes involved in melanocyte dendrite formulation and tyrosinase activity. Scott GA, J. Invest Dermatol. (2006); 126(4):855-61. The literature is, however, contradictory about the proteins involved in this process. See for example Park H.Y., J. Biol. Chem. (1999) 274:16470-8.

It is known that agents that induce tyrosinase activity or pigment production *in vitro* are not always able to induce skin tanning in humans upon topical treatment. For example, alpha-melanocyte-stimulating hormone (alpha-MSH) and its synthetic analogue Nle4DPhe7 alpha-MSH (NDP-MSH) induce dose-dependent increases in melanin content and in tyrosinase activity in the majority of human melanocyte cultures (Hunt G., Pigment Cell Res. (1994) 7(4):217-21). MSH and Nle4DPhe7 alpha-MSH are routinely used as research tools in melanogenesis studies. However, no effect has been documented by topical applications of MSH on human skin. Intravenous delivery, subcutaneous injections and oral delivery of MSH have been used in humans with different levels of success (E.g., Ugwu S.O., Biopharm Drug Dispos. (1997) 18(3):259-69). Similarly, MSH-analogs were shown to have limited or no topical delivery ability. MSH analogs used in clinical testing (e.g. Melanotan) are administered via injection.

The need for safe and effective topical treatments for skin darkening is recognized by the United States National Health Institute. As skin tanning is expected to reduce the risk of skin cancer, the National Cancer Institute (NCI) is supporting studies of topical agents that could induce skin darkening.

### SUMMARY OF THE INVENTION

In one aspect, the present invention features a method of darkening human skin, said method comprising topically applying to said skin a composition comprising a glycerophospholipid having a single fatty acid moiety.

The invention also relates to a method of darkening human skin, said method comprising topically applying a composition comprising a glycerophospholipid having a single fatty acid moiety to said skin daily for at least four weeks, such that said skin exhibits increased pigmentation compared with the same skin prior to application of said composition.

Finally, the invention also provides a composition comprising a glycerophospholipid having a single fatty acid moiety and cosmetically acceptable topical carrier.

In one embodiment, the glycerophospholipid having a single fatty acid moiety is present in the composition in a concentration of at least about 0.1% by weight, preferably at least about 1% by weight.

In another embodiment, the glycerophospholipid having a single fatty acid moiety is lysophosphatidylcholine (LPC).

Other features and advantages of the present invention will be apparent from the detailed description of the invention and from the claims.

### DETAILED DESCRIPTION OF THE INVENTION

It is believed that one skilled in the art can, based on the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

### Definitions

What is meant by "darkening the skin or hair" is darkening the appearance of human skin or hair, including, but not limited to, darkening human skin to either achieve a "sun tan" effect or to cover the light areas of the skin (e.g., as a result of a scar or a disease or a therapy) or darkening natural hair color or restoring discolored hair due to aging (e.g., gray or white hair) or external aggressions (e.g., excess exposure to sun or chlorine).

What is meant by a "product" is a product in finished packaged form. In one embodiment, the package is a container such as a plastic, metal or glass tube or jar containing the composition. The product may further contain additional packaging such as a plastic or cardboard box for storing such container. In one embodiment, the product contains instructions directing the user to apply the composition to the skin or hair to darken the skin (e.g., to tan the skin), even skin tone (e.g., to darken light areas of the skin or to treat or prevent mottled hyperpigmentation), or darken the hair (e.g., to darken light brown, blonde, gray or white hairs). Such instructions may be printed on the container, label insert, or on any additional packaging.

As used herein, "topically applying" means directly laying on or spreading on outer skin, scalp, or hair, e.g., by use of the hands or an applicator such as a wipe, roller, or spray.

As used herein, "cosmetically acceptable" means that the ingredients which the term describes are suitable for use in contact with tissues (e.g., the skin or hair) without undue toxicity, incompatibility, instability, irritation, allergic response, or the like.

As used herein, "safe and effective amount" means an amount of the composition sufficient to induce a darkening of human skin or hair, but low enough to avoid serious side effects. The safe and effective amount of the composition will vary with the area being treated, the age and skin type of the end user, the duration and nature of the treatment, the specific ingredient, or composition employed, the particular cosmetically acceptable carrier utilized, and like factors.

### Glycerophospholipid

Compositions of the present invention include a glycerophospholipid. As used herein "phospholipids" means a class of lipids comprising four components: fatty acids, a negatively charged phosphate group, alcohol with or without nitrogen, and a backbone. Phospholipids containing a glycerol backbone (HOCH₂- CH₂OH-CH₂ are known as glycerophospholipids or phosphoglycerides. Glycerophospholipids may have one, two or three fatty acid moieties. See, e.g., Biochemistry, Donald Voet and Judith G. Voet, John Wiley & Sons, Inc. p. 274.

The present invention relates to glycerophospholipids comprising a single fatty acid moiety; that is, one and only one fatty acid moiety is presented in the molecule. The present invention excludes phospholipids containing more than one fatty acid bonded to a backbone (e.g., phosphatidylcholine).

By "fatty acid moiety" it is meant a monobasic acid, especially those found in animal and vegetable fats and oils, having the general formula CₙH₂ₙ₊₁COOH. A fatty acid may be made up of saturated (e.g., caprylic, capric, lauric, myristic, palmitic, stearic, arachidic, lignoceric, etc.) or unsaturated aliphatic compounds (e.g., palmitoleic, oleic, linoleic, arachidonic, linoleic) with an even number of carbon atoms. The fatty acid may have at least about 8 carbon atoms, for example, from about 10 to about 24 carbon atoms, preferably from about 16 to about 20 carbon atoms.

The phosphorus-containing group of the glycerophospholipids is preferably a phosphate group. In a preferred embodiment, a phosphate group is bonded (e.g., via an ester linkage) to the glycerol backbone. The phosphate group is also preferably bonded to a polar group. The polar group may be, for example, but is not limited to choline (C₅ H₁₄NO), serine(C₃H₇NO₃), ethanolamine (C₂H₇NO), inositol (C₆H₁₂O₆), and the like.

In the most preferred embodiment, the glycerophospholipid having a single fatty acid moiety of this invention is lysophosphatidylcholine (LPC), a phospholipid containing one mole of fatty acid per mole of lipid in position sn-1. The chemical structure of LPC is shown below

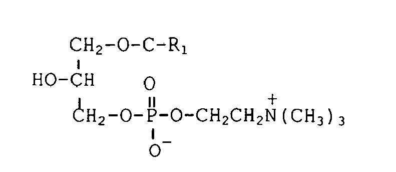

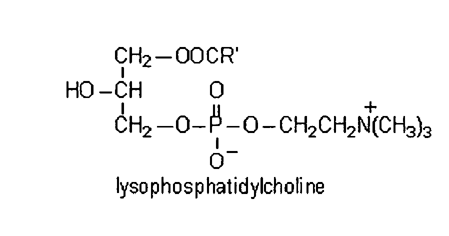

LPC may be isolated from animal (e.g. egg yolk, bovine brain), plant (e.g., soybean, sunflower and rapeseed), and microbial (e.g. Escherichia coli) sources (see, e.g., Leon F, J. Agric. Food Chem. 2004 Mar 10;52(5):1207-11 and Nestruck-Goyke A.C., Eur. J. Biochem. 1981 Feb;114(2):339-47) or may by chemically synthesized (Adlercreutz D, Biotechnol. Bioeng. 2002 May 20;78(4):403-11).

The above definition for glycerophospholipid having a single fatty acid moiety encompasses the components of what is traditionally referred to as "lysolecithin," a mixture of glycolipids, triglycerides, and lysophospholipids, but does not encompass the components of what is traditionally referred to as "lecithin," a mixture of glycolipids, triglycerides, and phospholipids(e.g., phosphatidylcholine, phosphatidylethanolamine, and phosphatidylinositol), or, as used in biochemistry, pure phosphatidylcholine.

### Other Darkening Agents

In one embodiment, the composition of the present invention further contains an additional darkening agent such as dihydroxyacetone, lawsone, erythulose, melanin, peptides, synthetic melanin derivatives, vanillin polymers, pigments, extracts such as but not limited to Coleus Forskoli extract, extracts from natural sources containing pigments (e.g., brown pigments from plants from the Hedychium genus or Bearberry genus or yellow, orange and red pigments from plants containing carotenoids or canthaxanthins); or synthetic chemicals such as compounds containing copper (e.g., copper salts such as CuCl₂) or synthetic carotenoids or canthaxantins. What is meant by an "extract" is a mixture of compounds isolated from a natural source (e.g., a plant).

Examples of synthetic melanin derivatives are disclosed in U.S. Patent Nos. 5,618,519, 5,384,116, and 5,227,459. Examples of soluble melanin derivatives are disclosed in 5,744,125, 5,225,435, 5,218,079, and 5,216,116. Examples of commercially available soluble melanin derivatives include Melasyn-100^{™} from San-mar laboratories, Inc. (Elmsford, NY) and MelanZe^{™} from Zylepsis (Ashford, Kent, United Kingdom).

These additional darkening agents will typically be present in the composition in an amount from about 0.001% to about 10% by weight.

In another embodiment, the composition may include a peptide. Examples of suitable peptides are described for example in US Patent No. 7,081,442 entitled "PEPTIDES AND THE USE THEREOF IN DARKENING THE SKIN" and represented by the formula below: 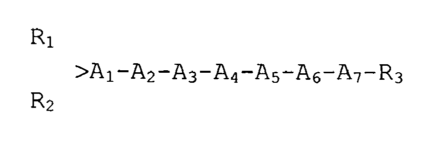 wherein:
A₁ is Ser or 2,3-diaP, or is absent;
A₂ is Val, Leu, Ile, or Cha;
A₃ is Val, Leu, Ile, or Cha;
A₄ is Gly or Ala;
A₅ is Lys, Arg, or Har;
A₆ is Val, Leu, Ile, or Cha, or is absent;
A₇ is Asp or Glu, or is absent; provided, A₇ is absent if A₆ is absent;
each R₁ and R₂, independently, is H, C₁₋₁₂ alkyl, C₇₋₁₀ phenylalkyl, or C (=O) E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino.

Other suitable peptides are disclosed in for example U.S Patent No. 6,797,697 entitled, "COMPOSITION CONTAINING A PEPTIDE AND A PIGMENT AND THE USE THERE OF IN DARKENING THE SKIN": 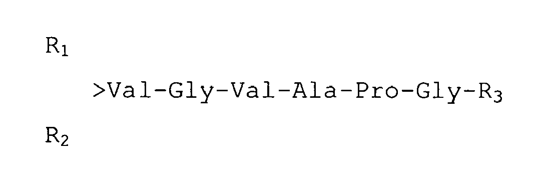 wherein:
each R₁ and R₂, independently, is H, C₁₋₁₂ alkyl, C₇₋₁₀ phenylalkyl, or C (=O) E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and
R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
or a cosmetically acceptable salt thereof;

U.S Patent No. 7,025,951 entitled, "COMPOSITION AND METHODS FOR DARKENING THE SKIN," also discloses suitable peptides of the following formula: 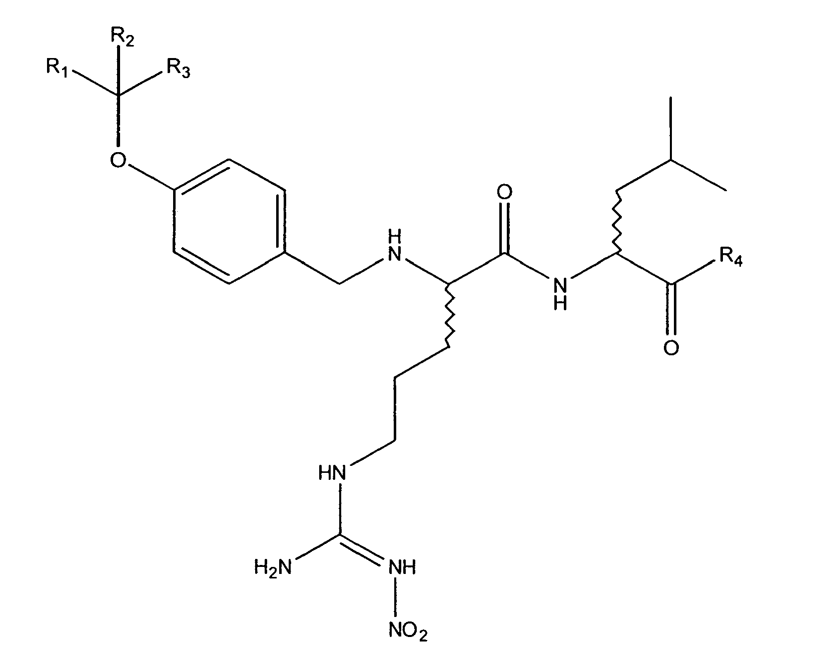
wherein R₁, R₂, and R₃, independently, are selected from the group consisting or H, Cl, or F; and
R₄ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
or a cosmetically acceptable salt thereof.

These peptides can be provided in the form of cosmetically acceptable salts. Examples of preferred salts are those with therapeutically acceptable organic acids, e.g., acetic, trifluoroacetic acid, palmitic, oleic, stearic, lactic, maleic, citric, malic, ascorbic, succinic, benzoic, salicylic, methanesulfonic, or pamoic acid, as well as polymeric acids such as tannic acid or carboxymethyl cellulose, and salts with inorganic acids such as the hydrohalic acids (e.g., hydrochloric acid), sulfuric acid or phosphoric acid.

The amount of peptide present in the composition will depend on the peptide used. The peptide typically will be present in the composition in an amount from about 0.001% to about 10% by weight, in particular in an amount from about 0.005% to about 5% by weight.

Methods for synthesizing such peptides are well documented and are within the ability of a person of ordinary skill in the art. See, e.g., Bodanszky M, Int. J. Pept. Protein Res. 25(5):449-74 (1985), Fmoc Solid Phase Peptide Synthesis, eds. Chan, W. & White, P. (Oxford University Press, 2000), and Chemical Approaches to the Synthesis of Peptides and Proteins, Lloyd-Williams, P. et al. (CRC Press, 1997).

### Topical Compositions

The compositions of the present invention are applied topically to human skin or hair. In one embodiment, the composition contains a safe and effective amount of (i) a glycerophospholipid having a single fatty acid moiety and (ii) a cosmetically acceptable topical carrier. In one embodiment, the cosmetically acceptable topical carrier is from about 50% to abut 99.99%, by weight, of the composition (e.g., from about 80% to about 99%, by weight, of the composition. In a preferred embodiment of the invention, the cosmetically acceptable topical carrier includes or consists essentially of water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limitative examples of such carriers. Other carriers can be formulated by those of ordinary skill in the art.

The topical compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol (200-600), polypropylene glycol (425-2025), glycerol, 1,2,4-butanetriol, sorbitol esters, 1,2,6-hexanetriol, ethanol, and mixtures thereof.

Topical compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin or hair. Examples of emollients include, but are not limited to, those set forth in the *International Cosmetic Ingredient Dictionary and Handbook,* eds. Pepe, Wenninger and McEwen, pp. 2930-36 (The Cosmetic, Toiletry, and Fragrance Assoc., Washington, D.C., 9^{th} Edition, 2002) (hereinafter "ICI Handbook").

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Although it is preferred that the composition of the present invention includes water, the composition may alternatively be anhydrous or an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, or a wipe containing powder).

It is also contemplated that the glycerophospholipid having a single fatty acid moiety may be formed into a liposome such as a unilamellar, multilamellar, and paucilamellar liposome. Such compositions can be prepared by combining a glycerophospholipid having a single fatty acid moiety and optionally other phospholipids, cholesterol and water. The liposome preparation may also be incorporated into one of the above topical carriers (e.g., a gel or an oil-in-water emulsion).

Micelle formulations are also useful compositions of the present inventions. Such micelle compositions are disclosed in the U.S. Patent No. 6,284,234.

Other encapsulation technologies are also useful in the compositions of the present invention, such as porous beads as those described in U.S. Patent Nos. 4,690,825 and 5,145,675.

The compositions useful in the subject invention may contain, in addition to the aforementioned components, a wide variety of additional oil-soluble materials and/or water-soluble materials conventionally used in compositions for use on skin and hair, at their art-established levels.

### Additional Cosmetically Active Agents

In one embodiment, the composition further contains another cosmetically active agent in addition to the glycerophospholipids having a single fatty-acid moiety. What is meant by a "cosmetically active agent" is a compound (e.g., a synthetic compound or a compound isolated from a natural source or a natural extract) that has a cosmetic or therapeutic effect on the skin or hair, including, but not limiting to, anti-acne agents, shine control agents, anti-microbial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, , *We cannot mix hair darkeners with hair removers, it is not logical... why to color the hair and then to remove it?* firming agents, anti-callous agents, and agents for hair and/or skin conditioning.

In one embodiment, the agent is selected from, but not limited to, the group consisting of hydroxy acids, benzoyl peroxide, D-panthenol, octyl methoxycinnimate, titanium dioxide, octyl salicylate, homosalate, avobenzone, carotenoids, free radical scavengers, spin traps, amines (e.g., DMAE and neutrol), retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides, amino acids such as proline, vitamins, lactobionic acid, acetyl-coenzyme A, niacin, riboflavin, thiamin, ribose, electron transporters such as NADH and FADH2, and other botanical extracts such as aloe vera, feverfew, oatmeal and derivatives and mixtures thereof. The cosmetically active agent will typically be present in the composition of the invention in an amount of from about 0.001% to about 20% by weight of the composition, e.g., about 0.005% to about 10% such as about 0.01% to about 5%.

Examples of vitamins include, but are not limited to, vitamin A, vitamin Bs such as vitamin B3, vitamin B5, and vitamin B12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

### Other Materials

Various other materials may also be present in the compositions useful in the subject invention. These include humectants, pH adjusters, chelating agents (e.g., EDTA), minerals, and preservatives (e.g., parabens). Examples of such agents are listed in pp. 2922-23, 2926-28, and 2892 of the ICI Handbook. In addition, the compositions useful herein can contain conventional cosmetic adjuvants, such as dyes, opacifiers (e.g., titanium dioxide), and fragrances.

The composition and formulations and products containing such compositions of the present invention may be prepared using methodology that is well known by an artisan of ordinary skill.

The following non-limiting example further illustrates the invention.

### Example: LPC induces skin darkening

The following preparations were made.

LPC preparation 1: Egg yolk lysophosphatidylcholine lipid powder was produced from chicken egg yolk by hydrolyzing with phospholipase A, extracting with organic solvents and refining. The lysophosphatidylcholine (99%, containing primarily palmitic and stearic acids) was obtained from Sigma (St. Louis, MO).

LPC preparation 2: Lysophosphatidylcholine (also called LPC-1) was purchased from Q.P. Corp. (Hyogo, Japan).

PC preparation (preparation 3): Phosphatidylcholine (Lecinol S-10, Hydrogenated lecithin from soy, containing 35% phosphatidylcholine) was obtained from Barnet (Englewood Cliffs, NJ).

All the phospholipids were dissolved in an ethanol/propylene glycol vehicle (70/30, v/v) at the concentration of 1% of solid phospholipid powder.

Preparations 1, 2 and 3 were tested for their ability to induce skin darkening in one human subject, with informed consent. One female volunteer, 50 years old, Fitzpatrick skin type II, with no history of skin cancer, no skin disease or unusual skin reaction, and no topical treatments on the study sites (the upper and lower parts of the arm) within a period of 30 days before enrollment and during the treatment period, was treated with preparations 1-3. Different preparations were used in four different studies. Two studies used the upper part of the arm, and two other studies used the lower part of the arm, which were treated twice a day for six to eight weeks with 10 µl of test phospholipid solutions (1%) in ethanol: propylene glycol (70:30, v/v) vehicle, or with vehicle alone. No other products were used and no sun exposure occurred during the treatment period.

Skin darkening was evaluated visually, and in some cases also by using a chromameter (ChromaMeter, Minolta, Osaka, Japan) and DRS (see Nguyen B.C., Br. J. Dermatol. 2003, 199(2):332-40). Measurements were taken at 4 and 8 weeks. The DRS instrument was assembled using a tungsten halogen light source (Model LS-1, Ocean Optics, Dunedin, Florida, USA), Miniature Fiber Optic Spectrometer (Model USB2000, Ocean Optics, Dunedin, Florida, USA) and custom-designed bifurcated fiber optic probe (Multimode Fiber Optics Inc, East Hanover, NJ, USA).

The scale used for reporting visual skin darkening is defined in the Table 1.

**Table 1**

| **SKIN DEPOSITION** | **DESCRIPTION** |
|---|---|
| ++ | Obvious visible tan-like color |
| + | Weak visible tan-like color |
| 0 | No visible tan-like color |

The visual tan-like color was somewhat stronger on the upper arm, relative to lower arm, possibly because of slightly darker baseline color of the lower arm than the upper arm. The visual results are summarized in Table 2, including the fading time of the darker color, when treatment was terminated at 8 weeks and skin color was followed for additional three weeks.

**Table 2**

| ACTIVE AGENT | 2 WEEKS | 4 WEEKS | 8 WEEKS | *8 WKS + 1 WK 2 | *8 WKS + WKS 3 | *8 WKS + WKS |
|---|---|---|---|---|---|---|
| Preparation 1 | + | ++ | ++ | ++ | + | 0 |
| Preparation 2 | + | +/++ | ++ | ++ | + | 0 |
| Preparation 3 | 0 | 0 | 0 | 0 | 0 | 0 |
| Vehicle | 0 | 0 | 0 | 0 | 0 | 0 |

| | | | | | | |
|---|---|---|---|---|---|---|
| * 8 weeks topical treatment followed by 1, 2 or 3 weeks without any treatment. | | | | | | |

These results show that LPC (preparations 1, 2), but not phosphatidylcholine (PC, preparation 3) induced visible skin darkening. The visible skin darkening induced by LPC did not change with routine daily activities (e.g., after water and soap washing). The darkening remained visible for at least 14 days after the end of the topical treatment, and started to fade between 14-20 days after termination of topical treatment.

Table 3 shows the DRS and chromameter results at 4 weeks of treatment. DRS confirmed the induction in melanin deposition (increased in DRS measurements) from both preparations 1 and 2, which correlated with the visual observations. The chromameter measurements reflected some variability in accordance with that methodology.

**Table 3**

| Active agent | Melanin (DRS, arbitrary units) | L* (Chromameter) |
|---|---|---|
| Vehicle | 1.248 | 67.28 |
| Preparation 1 | 1.274 | 68.27 |
| Preparation 2 | 1.304 | 67.69 |

This data suggest that, unexpectedly, the topical application of LPC, but not other phospholipids, induce visible skin darkening.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

### Preferred embodiments of the invention include:

1. A method of darkening human skin, said method comprising topically applying to said skin a composition comprising a glycerophospholipid having a single fatty acid moiety.
2. The method of embodiment 1, wherein said glycerophospholipid having a single fatty acid moiety is lysophosphatidylcholine.
3. The method of embodiment 1, wherein said glycerophospholipid having a single fatty acid moiety is present in said composition in a concentration of at least about 0.1% by weight.
4. The method of embodiment 1, wherein said glycerophospholipid having a single fatty acid moiety is present in said composition in a concentration of at least about 1% by weight.
5. The method of embodiment 1, wherein said composition further comprises an additional darkening agent selected from the group consisting of dihydroxyacetone, lawsone, erythulose, melanin, synthetic melanin derivatives, vanillin polymers, pigments, amines, peptides,) extracts and synthetic chemicals.
6. The method of embodiment 1, wherein said composition further comprises a peptide.
7. The method of embodiment 6, wherein the peptide has the formula: 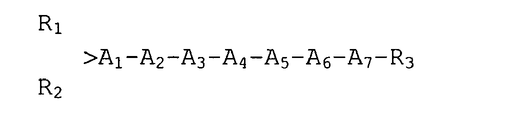 wherein:
   A₁ is Ser or 2,3-diaP, or is absent;
   A₂ is Val, Leu, Ile, or Cha;
   A₃ is Val, Leu, Ile, or Cha;
   A₄ is Gly or Ala;
   A₅ is Lys, Arg, or Har;
   A₆ is Val, Leu, Ile, or Cha, or is absent;
   A₇ is Asp or Glu, or is absent; provided, A₇ is absent if A₆ is absent;
   each R₁ and R₂, independently, is H, C₁₋₁₂ alkyl, C₇₋₁₀ phenylalkyl, or C (=O) E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino.
7b. The method of embodiment 6, wherein the peptide has the formula: 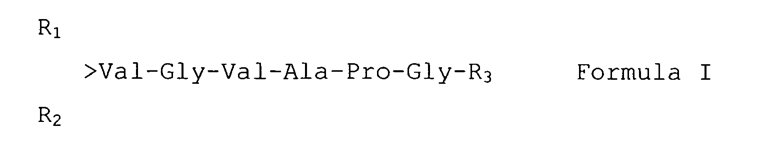 wherein:
   each R₁ and R₂, independently, is H, C₁₋₁₂ alkyl, C₇₋₁₀ phenylalkyl, or C (=O) E₁, where E₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and
   R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
   or a cosmetically acceptable salt thereof.
7c. The method of embodiment 6, wherein the peptide has the formula: 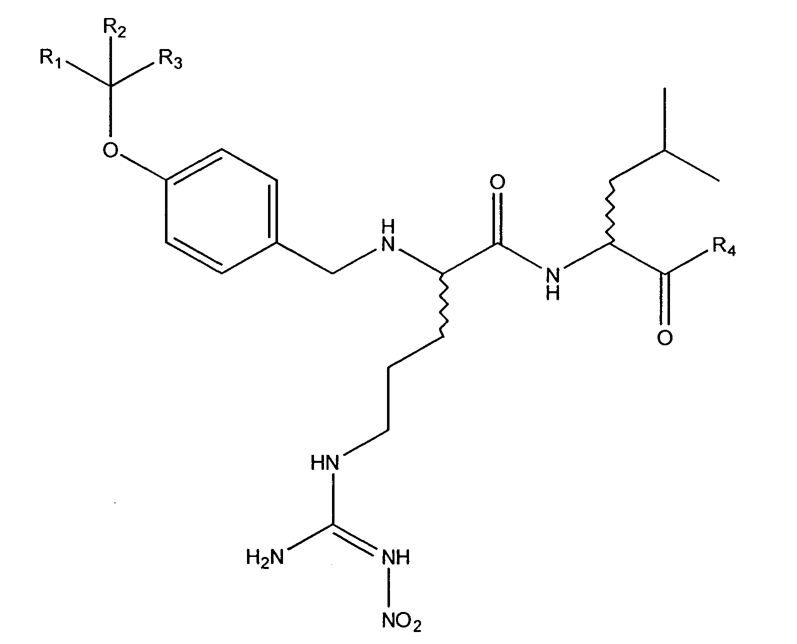
   wherein R₁, R₂, and R₃, independently, are selected from the group consisting or H, Cl, or F; and
   R₄ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
   or a cosmetically acceptable salt thereof.
7. The method of embodiment 1, wherein said composition further comprises an extract.
8. The method of embodiment 1, wherein said composition further comprises an additional agent selected from the group consisting of peptides, extracts, pigments, antioxidants, DHA, amines, preservatives, and cosmetically acceptable carriers.
9. A method of darkening human skin, said method comprising topically applying a composition comprising a glycerophospholipid having a single fatty acid moiety to said skin daily for at least four weeks, such that said skin exhibits increased pigmentation compared with the same skin prior to application of said composition.
10. The method of embodiment 9, wherein said glycerophospholipid having a single fatty acid moiety is lysophosphatidylcholine.
11. The method of embodiment 9, wherein said glycerophospholipid having a single fatty acid moiety is present in said composition in a concentration of at least about 0.1% by weight.
12. The method of embodiment 9, wherein said glycerophospholipid having a single fatty acid moiety is present in said composition in a concentration of at least about 1% by weight.
13. The method of embodiment 9, wherein said composition further comprises an additional darkening agent selected from the group consisting of dihydroxyacetone, lawsone, erythulose, melanin, synthetic melanin derivatives, vanillin polymers, pigments, amines, peptides, extracts and synthetic chemicals.
14. The method of embodiment 9, wherein said composition further comprises a peptide.
15. The method of embodiment 9, wherein the peptide has the formula: 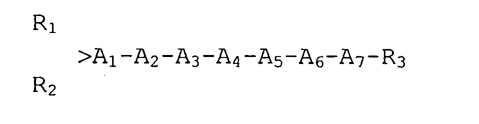 wherein:
   A₁ is Ser or 2,3-diaP, or is absent;
   A₂ is Val, Leu, Ile, or Cha;
   A₃ is Val, Leu, Ile, or Cha;
   A₄ is Gly or Ala;
   A₅ is Lys, Arg, or Har;
   A₆ is Val, Leu, Ile, or Cha, or is absent;
   A₇ is Asp or Glu, or is absent; provided, A₇ is absent if A₆ is absent;
   each R₁ and R₂, independently, is H, C₁₋₁₂ alkyl, C₇₋₁₀ phenylalkyl, or C (=O) E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino.
15b. The method of embodiment 14, wherein the peptide has the formula: 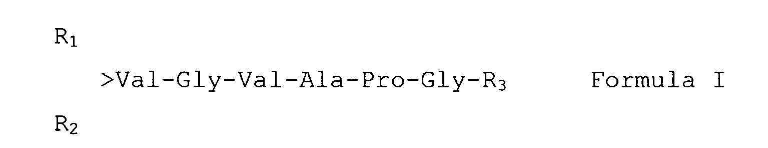 wherein:
   each R₁ and R₂, independently, is H, C₁₋₁₂ alkyl, C₇₋₁₀ phenylalkyl, or C (=O) E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and
   R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
   or a cosmetically acceptable salt thereof.
15c. The method of embodiment 14, wherein the peptide has the formula: 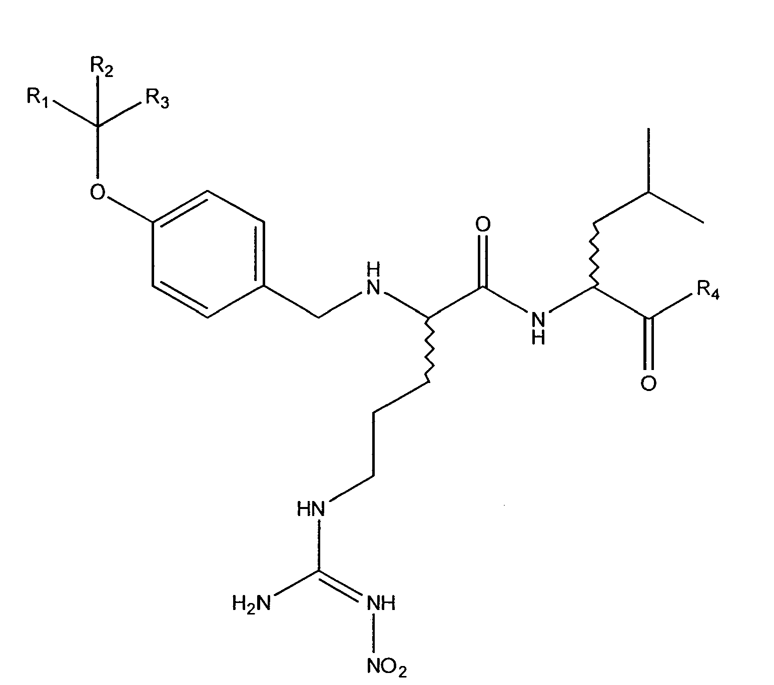
   wherein R₁, R₂, and R₃, independently, are selected from the group consisting or H, Cl, or F; and
   R₄ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
   or a cosmetically acceptable salt thereof.
16. The method of embodiment 9, wherein said composition further comprises an extract.
17. The method of embodiment 9, wherein said composition further comprises an additional agent selected from the group consisting of peptides, extracts, pigments, antioxidants, DHA, preservatives, amines, and cosmetically acceptable topical carriers.
18. A composition comprising a glycerophospholipid having a single fatty acid moiety and cosmetically acceptable topical carrier.
19. The composition of embodiment 18, further comprising an additional darkening agent selected from the group consisting of dihydroxyacetone, lawsone, erythulose, melanin, synthetic melanin derivatives, vanillin polymers, pigments, peptides, amines, extracts and synthetic chemicals.
20. The composition of embodiment 19, wherein said additional darkening agent is a peptide.
21. The composition of embodiment 20, wherein the peptide has the formula: 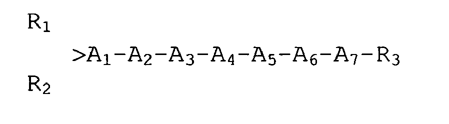 wherein:
   A₁ is Ser or 2,3-diaP, or is absent;
   A₂ is Val, Leu, Ile, or Cha;
   A₃ is Val, Leu, Ile, or Cha;
   A₄ is Gly or Ala;
   A₅ is Lys, Arg, or Har;
   A₆ is Val, Leu, Ile, or Cha, or is absent;
   A₇ is Asp or Glu, or is absent; provided, A₇ is absent if A₆ is absent;
   each R₁ and R₂, independently, is H, C₁₋₁₂ alkyl, C₇₋₁₀ phenylalkyl, or C (=O) E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C ₇₋₁₀ phenylalkyl; provided that when either R₁ or R₂ is C (=O) E₁, the other must be H; and R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino.
22b. The composition of embodiment 20, wherein the peptide has the formula: 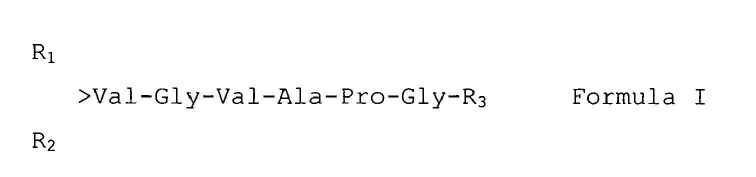 wherein:
   each R₁ and R₂, independently, is H, C₁₋₁₂ alkyl, C₇₋₁₀ phenylalkyl, or C(=O)E₁, where E ₁ is C₁₋₂₀ alkyl, C₃₋₂₀ alkenyl, C₃₋₂₀ alkynyl, phenyl, 3,4-dihydroxyphenylalkyl, naphthyl, or C₇₋₁₀ phenylalkyl; provided that when either R₁ or R₂ is C (=O) E₁, the other must be H; and
   R ₃ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
   or a cosmetically acceptable salt thereof.
22c. The composition of embodiment 20, wherein the peptide has the formula: 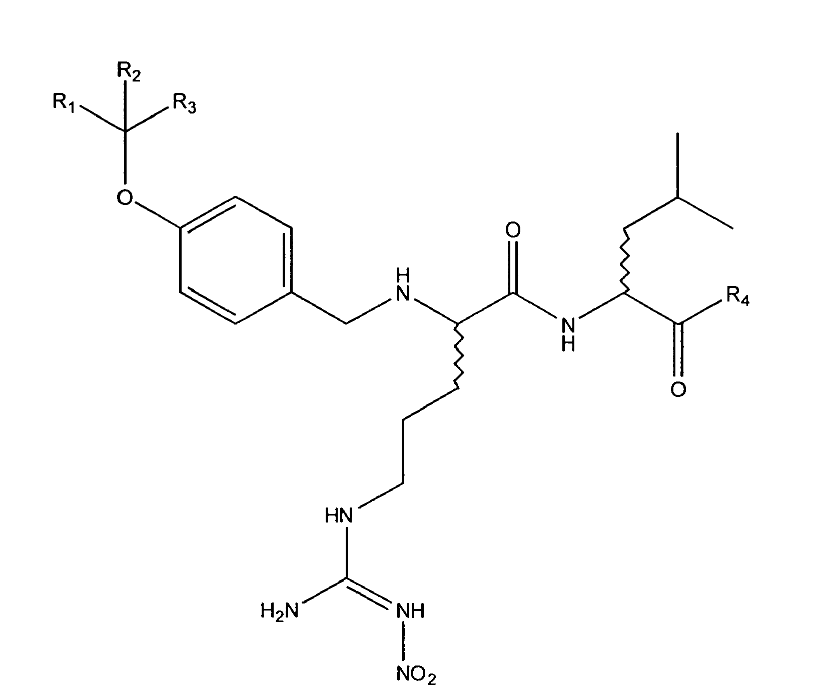
   wherein R₁, R₂, and R₃, independently, are selected from the group consisting or H, Cl, or F; and
   R₄ is OH, NH₂, C₁₋₁₂ alkoxy, C₇₋₁₀ phenylalkoxy, C₁₁₋₂₀ naphthylalkoxy, C₁₋₁₂ alkylamino, C₇₋₁₀ phenylalkylamino, or C₁₁₋₂₀ naphthylalkylamino;
   or a cosmetically acceptable salt thereof.
22. The composition of embodiment 20, wherein said additional darkening agent is an extract.
23. A composition comprising a glycerophospholipid having a single fatty acid moiety, and an additional agent selected from the group consisting of peptides, extracts, pigments, antioxidants, DHA, preservatives, amines, and cosmetically acceptable topical carriers.

## Claims

1. A method of darkening human skin, said method comprising topically applying to said skin a composition comprising a glycerophospholipid having a single fatty acid moiety.

2. The method of claim 1, wherein said glycerophospholipid having a single fatty acid moiety is lysophosphatidylcholine.

3. The method of claim 1, wherein said composition further comprises an additional darkening agent selected from the group consisting of dihydroxyacetone, lawsone, erythulose, melanin, synthetic melanin derivatives, vanillin polymers, pigments, amines, peptides,) extracts and synthetic chemicals.

4. A method of darkening human skin, said method comprising topically applying a composition comprising a glycerophospholipid having a single fatty acid moiety to said skin daily for at least four weeks, such that said skin exhibits increased pigmentation compared with the same skin prior to application of said composition.

5. The method of claim 4, wherein said glycerophospholipid having a single fatty acid moiety is lysophosphatidylcholine.

6. The method of claim 4, wherein said glycerophospholipid having a single fatty acid moiety is present in said composition in a concentration of at least about 0.1% by weight.

7. The method of claim 4, wherein said composition further comprises a peptide.

8. The method of claim 4, wherein said composition further comprises an extract.

9. A composition comprising a glycerophospholipid having a single fatty acid moiety and cosmetically acceptable topical carrier.

10. A composition according to claim 9 further comprising an additional darkening agent selected from the group consisting of dihydroxyacetone, lawsone, erythulose, melanin, synthetic melanin derivatives, vanillin polymers, pigments, amines, peptides, extracts and synthetic chemicals.

11. A composition according to claim 10 wherein the additional darkening agent is an extract.
